# EUROPEAN PATENT SPECIFICATION

(11) **EP 2 883 529 B1**
(45) Date of publication and mention of the grant of the patent: **14.08.2019**
(21) Application number: 14161788.6
(22) Date of filing: 26.03.2014
(51) Int. Cl.: A61K 8/22, A61K 8/81, A61K 8/34, A61K 8/19, A61Q 11/02

(54) **Bleaching gel**
Bleichgel
Gel de blanchiment

(30) Priority: 10.12.2013 US 201361914051 P
(43) Date of publication of application: 17.06.2015
(73) Proprietor: Colgate-Palmolive Company, New York, NY 10022 (US)
(72) Inventor: Khawaled, Kamal, 20200 Shefaram (IL); Mushyakov, Tanya, 20692 Yokneam Illit (IL)
(74) Representative: Wibbelmann, Jobst

(56) References cited:
- WO-A1-2008/001388
- US-A1- 2009 117 513
- US-A1- 2011 189 637
- US-B1- 6 503 485
- DATABASE GNPD [Online] MINTEL; 29 September 2003 (2003-09-29), Den-Mat: "Rembrandt 2-Hour White Kit", XP002738809, Database accession no. 10149065
- DATABASE GNPD [Online] MINTEL; 30 June 2003 (2003-06-30), Den-Mat: "Rembrandt Improved Superior Whitening Kit", XP002738810, Database accession no. 10139984

## Description

### TECHNOLOGY FIELD

The present gel relates to the field of dental bleaching compositions and in particular to electrically conductive dental bleaching compositions.

### BACKGROUND

Stained or discolored teeth are considered as a cosmetic impairment. Teeth's whitening is a cosmetic treatment done to improve the appearance of teeth. Teeth are whitened to remove the effects of coffee, cigarettes, and other substances that permanently stain or discolor teeth. Different techniques of teeth whitening or bleaching are known and relatively simple procedures improving the stained teeth appearance exist. The procedures can be conducted in a dental Clinique, office or in a residential environment.

A variety of dental bleaching compositions and dental bleaching devices or trays exist on the market. Such dental bleaching compositions are typically applied to a person's teeth using a dental tray configured so as to retain the dental composition against the person's teeth. The treatment continues for about 10 minutes to few hours and although conducted in residential environment it is somehow reducing the freedom of speech, movement and operation of the treated person. In addition, it is not comfortable for the treated person to hold the dental tray in the mouth over relatively long periods of time.

Treated person comfort during the teeth bleaching treatment may be improved by shortening the time of bleaching. Most common bleaching agents are peroxides, including but not limited to hydrogen peroxide, carbamide peroxide, sodium perborate, and sodium percarbonate. The simplest way to shorten the treatment time is to increase the concentration of the peroxide. This can however, result in irritation and even some damage of gums or lips or other soft tissue.

Another way to bleach teeth more quickly is to speed up the chemical reaction related to the bleaching process by heat or light. Both heat and light alone or in combination are known to accelerate chemical reactions. Different bleaching compositions having increased bleaching activity have been developed for use in cooperation with heat or light.

WO 2008/001388 A1 discloses the electrochemically whitening of teeth whereby a metal salt solution is applied to the teeth, followed by applying an oxidizing agent to the teeth, and transmitting an electrical current to the teeth so as to activate and reduce the oxidizing agent for effecting whitening of the teeth.

### SUMMARY

It was the object of the present invention to provide a teeth bleaching gel method with a composition that is less irritating than prior art gels, is highly efficient, can bleach within shorter time and results in whitened teeth when applied. The present invention concerns a teeth bleaching method comprising applying to the teeth to be bleached a layer of gel, wherein the gel comprises A dental bleaching gel composition comprising (percentages w/w are based on the weight of the final bleaching gel composition): a peroxidehydrogen peroxide, wherein the amount of hydrogen peroxide is 1 to 35% w/w, or carbamide peroxide, sodium perborate, or sodium percarbonate as a peroxide releasing compound, wherein the amount of the peroxide releasing compound is 1 to 35% w/w; a cross-linked polyacrylate polymer, wherein the amount of cross-linked polyacrylate polymer is 1 to 5% w/w; an aqueous vehicle, wherein the amount of glycerine is 20 to 30% w/w; and/or wherein the amount of water is 25 to 50% w/w; and at least one gel electrical conductivity enhancing ingredient consisting of alkali nitrate in an amount of 1 to 5% w/w and/or alkali hydroxide in an amount of 1 to 5% w/w; wherein the electrical conductivity of the gel is at least 250 microsiemens/cm; and further applying to the teeth covered by the layer of gel with electrical conductivity higher than 250 microsiemens/cm electric current of at least 1 milliampere; and bleaching the teeth.

Other ingredients that are commonly used in bleaching gels, can be used in the composition of the present invention as well, in the amounts that are normally used, as long as such an ingredient is dentally acceptable. Examples are agents affecting antimicrobial and oxidizing properties of the gel, binding agents, mouth refreshing and flavouring components and components improving bleaching gel viscosity and wetting properties which are added as required to the composition.

### DESCRIPTION

The present invention provides a bleaching gel composition that is less irritating than prior art gels, is highly efficient, can bleach within short time and results in whitened teeth when applied only few times for some minutes, such as twice a week for only some minutes. The gel has a composition allowing it to penetrate deep within the enamel to erase most of embedded stains without sensitivity or irritation.

The favourable effect is achieved by using the components as defined in the claims. The gel of the present invention when activated by a micro-current is highly efficient.

### Definitions

In the context of the present disclosure the terms "dental bleaching" and "dental whitening" have the same meaning.

A peroxide can be any known peroxide that is compatible with the human skin and mucous. Hydrogen peroxide is one example.

A "peroxide releasing compound" comprises compounds that when activated by a micro-current release peroxide. Examples are carbamide peroxide, sodium perborate, or sodium percarbonate.

The term "micro-current" when used in this application refers to current of 25 milliampere or less.

Electric current is known to accelerate the bleaching reaction. The electric current is activating the bleaching agent and accelerating the bleaching reaction. The current activates certain ingredients of a specially formulated whitening gel, allowing it to penetrate deep within the enamel to erase most of embedded stains without sensitivity or irritation. Use of electrical current and specially formulated gel has proved to deliver whitening results much better than light or heat activated teeth whitening treatments.

The present invention allows to use micro-current for activating the bleaching agent. The term "micro-current" when used in this application has its common meaning and particularly refers to a current of 25 milliampere or less, in particular 15 milliampere or less, preferably 1 to 10 milliampere. Application of electric current to the bleaching gel composition is less expensive than application of light generated by an assembly of LEDs and or heat. Electric current is also simpler and easier to control and regulate. The dental bleaching composition of the present invention with certain level of electric conductivity allows the use of electric current to activate the bleaching agent and accelerate the bleaching reaction.

It has been found that when using a composition having a certain degree of electric conductivity as outlined below, activation can occur using micro-current.

Although bleaching gels are known that comprise ionic compounds, their electric conductivity is not sufficient to produce an electric current supporting activation of the bleaching agent and acceleration of the bleaching reaction. Currently available on the market dental bleaching compositions are either electrically non-conductive or have very low conductivity typically below 200 microsiemens/cm.

A bleaching gel as described herein having an electric conductivity of at least 250 microsiemens/cm can be activated by a low current, in particular by a micro-current to become efficient enough to allow teeth whitening even with application for a short time.

A dental bleaching gel composition used with the present invention and having a significantly higher electric conductivity can support faster development and commercialization of dental bleaching devices using electric current to accelerate the bleaching reaction. Such dental bleaching gels and devices or trays can reduce the cost of dental bleaching and are more effective in whitening or bleaching teeth than existing products currently available on the market.

When micro-current is applied to the gel this will create peroxide radicals that are then available for the bleaching reaction.

The dental bleaching gel compositions can further include a number of ingredients that are typically present in this type of compositions and in particular, apart from a bleaching agent, a thickening agent, and a vehicle can include a liquid component, a basic component, a flavoring component, and some other components as it could be desired to achieve a particular dental bleaching gel composition properties. For example, the composition could further include light sensitive or heat sensitive ingredients that upon action of light or heat will accelerate the teeth bleaching process.

The dental bleaching gel ingredients listed in different examples follow the International Nomenclature of Cosmetic Ingredients, abbreviated (INCI).

Whenever amounts of ingredients are mentioned in percent, these refer to % w/w, i.e. weight of the ingredient per weight of the final composition.

An essential ingredient of the present bleaching gel is a peroxide or a peroxide releasing agent as bleaching agent; examples are hydrogen peroxide, or a sodium perborate, or sodium percarbonate. The bleaching agent is used in an amount that provides at least some whitening of teeth but less than an amount that irritates or hurts the tissue or mucous membrane. The amount of bleaching agent can be in a range of 1 to 35 % w/w, in particular 3 to 10 % w/w or 6 to 9 % w/w peroxide, based on the weight of the final bleaching gel composition.

Another essential ingredient is a thickening agent. Thickening agents for preparing bleaching gels are known and can be used for the present composition. The thickening agent is used to adapt the rheology/viscosity of the gel such that it can be applied easily and does not flow away when applied. An example for a thickening agent is a cross-linked polyacrylate. The thickening agent is used in an amount that provides for the desired viscosity of the gel, the skilled artisan can find a suitable amount in known manner. A useful amount is in the range of 1 to 5 % w/w.

Thickening agent gel component is a rheology modifier that increases the viscosity of the dental bleaching composition to prevent runoff of the dental bleaching gel composition out of the dental tray. Increased viscosity of the dental bleach composition is also useful in reducing contact with soft tissues such as gums, lips and some internal surface of the mouth tissue. In one example, a cross-linked polyacrylate polymer commercially available from the Lubrizol Corporation Wickliffe, Ohio 44092 USA under trade name Carbopol 940 or 980 in concentration of 1% to 5% w/w is used as a thickening agent.

Furthermore, the bleaching gel comprises an aqueous vehicle that preferably includes at least one alcohol, such as glycerol, a glycol, or a mixture thereof. Examples for a vehicle are a mixture of water and glycerol and/or a glycol. The glycol can be propylene glycol. In one embodiment the vehicle is a mixture of water and glycerol, glycerol improves wetting of the tooth surface and, thus, facilitates application of the bleaching gel.

The aqueous vehicle is preferably present in amount of 45 to 85 % w/w, where 20 to 35 % w/w can be an alcoholic component, such as glycerol or glycol.

Another essential ingredient is an electrical conductivity enhancing compound or electrolyte, such as a salt or a base. Examples for electrical conductivity enhancing compounds are an alkali hydroxide, such as potassium hydroxide, or an alkali nitrate, such as potassium nitrate, or a mixture thereof. A combination of potassium hydroxide and potassium nitrate has been found particularly valuable. As the bleaching gel is applied to mucous, the amount of alkali hydroxide should not be too high to not irritate the mucous. An amount that results in a pH in the range of 5 to 7 has been found useful. Other ingredients could be added to adapt the pH if necessary.

In one example, the bleaching gel could include an electrical conductivity enhancing ingredient or a number of ingredients. The electrical conductivity enhancing ingredient could be electrolytes, such as for example, potassium nitrate or potassium hydroxide in different amounts or percentage. A mix of both of them could also be used to enhance conductivity of the dental bleaching gel composition. Potassium nitrate could also be used to lower the tooth sensitivity and the potassium hydroxide could be used to adjust the bleaching gel pH. Electric current could be used to accelerate destabilization of the peroxides and facilitate release of oxygen ions and oxygen radical's derivative participating among others in the process of the dental bleaching. Potassium hydroxide concentration in the dental bleach composition could be 1% to 5% by weight. And the potassium nitrate concentration in the dental bleach composition could be 1% to 5% by weight.

In one embodiment of the bleaching gel a bleaching agent comprising hydrogen peroxide (H₂Cl₂) is used in combination with potassium hydroxide (KOH). The concentration of hydrogen peroxide is then 1% to 35% by weight, based on the weight of the final bleaching gel, and the concentration of potassium hydroxide used as a 50% solution in D.I water could be 1% to 5% by weight. Hydrogen peroxide is commercially available from a number of suppliers, for example, from Sigma-Aldrich Co. LLC, Missouri 63031 USA.

According to some examples, glycerin and water are used as liquid components in the present dental bleaching gel composition. Glycerin is also used as a humectant facilitating better wetting of teeth surface by the dental bleaching gel composition. In some examples, glycerin is included in a concentration of 20% to 35% w/w and water is included in a concentration of 25% to 50% w/w.

For stabilization the bleaching gel can include further ingredients. Binding agents like polyvinyl pyrrolidone have been found useful, they can be used in an amount of 0.5 to 2 % w/w. Moreover, complexing agents such as disodium ethylene diamine tetraacetic salt can be used to improve stability of the gel. An amount of 0.1 to 1 % w/w has been found suitable.

To improve the taste of the dental bleaching gel composition a flavoring agent or a sweetener could be added to the composition. Examples of such sweeteners could include saccharin sodium salt hydrate in concentration of 0.1% to 0.3%. Flavorings could include mint and peppermint oil in concentration of 0.1% to 0.3% and the like. Both flavorings are commercially available, for example from Sigma-Aldrich Co. LLC, Missouri 63031 USA.

The gel can additionally include heat or light sensibilizers to further increase the activity.

The bleaching gel is used for bleaching teeth by application of a micro-current. Another aspect of this application is thus a method for bleaching teeth comprising:
applying to the teeth to be bleached a layer of gel with electrical conductivity higher than 250 microsiemens/cm;
applying to the teeth covered by a layer of gel with electrical conductivity higher than 250 microsiemens/cm electric current of at least 1 milliampere; and
bleaching the teeth.

It has been found that by using the bleaching gel as characterized in the claims and a low current of about 1 to 10 milliampere teeth can be bleached within minutes, for example within 5 to 10 minutes. The gel is not irritating and the low current is not harmful or unpleasant for the user.

### Dental bleaching gel application and testing procedure

The dental bleaching gel as described above is used for bleaching teeth in combination with a micro-current device that provides current for the activiation of peroxide. A useful micro-current device that can be used with the gel of the present application is Pearl™ dental bleaching tray, commercially available from Syneron Beauty Ltd., Yoqneam 2069202 Israel. This bleaching tray was used for testing the bleaching gel:
a. The Pearl™ dental bleaching tray commercially available from Syneron Beauty Ltd., Yoqneam 2069202 Israel was filled in with the present gel in an amount sufficient to form layer of gel on at least the surface of the teeth to be bleached.
b. The tray with the gel was placed in the mouth and electric current supply was activated for 5 minutes.
c. At the end of the treatment, the remaining gel was removed by washing the teeth.

Further embodiments are outlined in the following examples.

### EXAMPLE 1 DENTAL BLEACHING GEL

A gel comprising 6 % hydrogen peroxide was prepared using the following ingredients:

| Ingredient | Concentration (%) w/w |
|---|---|
| Glycerin | 25-30 |
| Carbopol 940/Carbopol 980 | 1-5 |
| Hydrogen Peroxide 30% | 20-25 |
| Deionized Water | 40-50 |
| Potassium Nitrate | 2-3 |
| Potassium Hydroxide (50% in water) | 2-3 |
| PVP(Polyvinylpyrrolidone) | 0.5-2 |
| EDTA (Disodium Ethylenediaminetetraacetic salt) | 0.1-1 |
| Saccharin sodium salt hydrate | 0.2 |
| Peppermint oil | 0.2 |

120 gram of the 6% HP dental gel bleaching composition were prepared by combining the following ingredients according to the following procedure:
3.6g of potassium nitrate were mixed with 0.24g of saccharine, 0.6g EDTA and dissolved at room temperature in 40 ml of water. Two to three drops of KOH where added to the mixture.

A mixer was used to stir the mixture for about 5-10 (five-ten) minutes. At the beginning of the stirring 3 grams of Carbopol® (cross-linked polyacrylate polymer) was added to the mixture. The mixture was stirred at a low speed of 200 - 250 RPM.

After about 5-10 minutes of stirring the mixture, 40 ml (32g) of glycerol were added to the mixture and the stirring continued for another 5-10 minutes at a speed of 500-700 RPM.

Following this 24 ml of hydrogen peroxide (30%W/W solution) were added to the mixture and the mixture was stirred for another 5-10 minutes at a speed of 500-700 RPM.

Three (3) milliliter of potassium hydroxide 50%W/W solution where added to the mixture and the mixture stirring continued for another five (5-10) minutes at a speed of 750- 1000 RPM.

Peppermint oil in amount of 0.2% w/w was added to the mixture and stirred for about a minute at a speed of 1000 RPM.

PVP (Polyvinylpyrrolidone) in an amount of 0.6g was added to the almost ready composition and stirred for another 10 minutes at a speed of 1000 RPM.

The process produced about 120 g of ready to use gel composition. The produced dental bleaching gel composition was stable and was able to adhere and retain in a dental tray in place against a person's teeth. The dental bleaching composition had a conductivity of 500 - 700 microsiemens/cm.

Pearl™ dental tray was used to test the bleaching properties of the bleaching gel composition of Example 1. Application of electric current of about 6 milliampere for 5 minutes bleached or whitened the teeth. The result was compared visually to teeth bleaching procedure using the Pearl™ teeth whitening system and commercially available teeth bleaching gels with electrical conductivity below 200 microsiemens/cm. The current teeth bleaching gel was found to result in two or three additional whiting shades as compared to the teeth bleaching performed by the Pearl™ system for 5 minutes using commercially available teeth bleaching gel.

Gel composition conductivity was measured by Digital Conductivity Meter model Traceable 4063, manufactured by Control Company, Friendswood, TX 77546, USA, (www.control3.com). The measurement was performed by a probe that contained platinum electrodes and a thermistor.

### EXAMPLE 2 DENTAL BLEACHING GEL

| Ingredient | Concentration w/w (%) |
|---|---|
| Glycerin/propylene glycol | 25-30 |
| Carbopol | 1-5 |
| Hydrogen Peroxide 30% w/w | 25-35 |
| Deionized Water | 30-40 |
| Potassium Nitrate | 2-3 |
| Potassium Hydroxide (50% in water) | 2-3 |
| PVP(Polyvinylpyrrolidone) | 0.5-2 |
| EDTA (Disodium Ethylenediaminetetraacetic salt) | 0.1-1 |
| Saccharin sodium salt hydrate | 0.2 |
| Peppermint oil | 0.2 |

In Example 2, 9% HP w/w gel dental bleaching composition was prepared using the following ingredients and according to the following procedure:
3.6g of potassium nitrate were mixed with 0.24g saccharine, 0.6g EDTA and dissolved at room temperature in 42 ml of water. Two or three drops of KOH were added to the mixture.

A mixer was used to stir the mixture for about 5-10 minutes. At the beginning of the stirring 3 grams of Carbopol® were added to the mixture. The mixture was stirred at a low speed of 200 - 250 RPM.

After about 5-10 minutes of stirring the mixture, 40 ml (32g) of glycerol were added to the mixture and the stirring continued for another 5-10 minutes at a speed of 500 - 700 RPM.

Following this 36 ml of hydrogen peroxide (30% solution) were added to the mixture and the mixture was stirred for another 5-10 minutes at a speed of 500 - 700 RPM.

Three (3) milliliter of potassium hydroxide 50% w/w solution where added to the mixture and the mixture stirring continued for another five (5 - 10) minutes at a speed of 750 - 1000 RPM.

Peppermint oil in amount of 0.2% w/w was added to the mixture and stirred for about a minute at a speed of 1000 RPM.

PVP (Polyvinylpyrrolidone) in an amount of 0.6g was added to the almost ready composition and stirred for another 10 minutes at a speed of 1000 RPM.

The process produced about 120 g of ready to use gel composition. The produced dental bleaching gel composition was stable and was able to adhere and retain in a dental tray in place against a person's teeth. The dental bleaching composition had a conductivity of 250 - 500 microsiemens/cm. Syneron Medical Ltd., Pearl™ dental tray was used to test the bleaching properties of the bleaching gel composition of Example 2. Application of electric current of about 10 milliampere for 5 minutes bleached or whitened the teeth. The result was compared visually to teeth bleaching procedure using the Pearl™ teeth whitening system and the current gel and commercially available teeth bleaching gels with electrical conductivity below 200 microsiemens/cm. The current gel was found to be superior to the commercially available teeth bleaching gels and resulted in 3 to 4 additional white shades as compared to teeth bleaching performed by using the Pearl™ tray with commercially available teeth bleaching gel for 5 minutes.

Gel composition conductivity was measured by Digital Conductivity Meter model Traceable 4063, manufactured by Control Company, Friendswood, TX 77546, USA, (www.control3.com). The measurement was performed by a probe that contained platinum electrodes and a thermistor.

### EXAMPLE 3 DENTAL BLEACHING GEL

| Ingredient | Concentration (%) w/w |
|---|---|
| Glycerin /propylene glycol | 20 - 30 |
| Carbopol 940/ Carbopol 980 | 1 - 5 |
| Hydrogen Peroxide 30% | 25 - 35 |
| Deionized Water | 30 - 40 |
| Potassium Nitrate | 2 - 3 |
| Sodium fluoride | 0.1 - 1.0 |
| Potassium hydroxide 30%w/w | 2 - 3 |
| PVP (Polyvinylpyrrolidone) | 0.5 - 2.0 |
| EDTA (Disodium Ethylenediaminetetraacetic salt) | 0.1 - 1 |
| Saccharin sodium salt hydrate | 0.2 |
| Peppermint oil | 0.2 |

A dental bleaching composition was prepared by combining the ingredients (in weight percent) as shown in the above table.

The procedure produced about 120 g of ready to use gel composition. The produced dental bleaching composition was stable and was able to adhere and retain a dental tray in place against a person's teeth. The dental bleaching composition had a conductivity of 800 - 1000 microsiemens/cm. Pearl™ dental tray was used to test the bleaching properties of the bleaching gel composition of Example 3. Application of electric current of about 10 milliampere for 5 minutes bleached or whitened the teeth. The result was compared visually to teeth bleaching procedure using the Pearl™ tray with commercially available teeth bleaching gels with electrical conductivity below 200 microsiemens/cm . The results of the teeth bleaching using the present gel were found to be superior to teeth bleaching performed by the Pearl™ system for 5 minutes using the commercially available teeth bleaching gels with low electric conductivity.

Gel composition conductivity was measured by Digital Conductivity Meter model Traceable 4063, manufactured by Control Company, Friendswood, TX 77546, USA, (www.control3.com). The measurement was performed by a probe that contained platinum electrodes and a thermistor.

### EXAMPLE 4 Comparative Teeth Whitening Test Results

In order to compare electric current assisted teeth whitening method efficacy with the non-current assisted teeth whitening methods a test procedure was set. The experimental set-up included a mock-up of twenty teeth stained to different levels of shades. The mock-up teeth were cut perpendicular to their long axis and divided into a total of forty mock-up teeth. Than the prepared specimens were divided into two groups; Group A and Group B with each group including twenty teeth with shades identical to the shades of the second group.

Twenty teeth of Group A were treated using the present electrically conductive whitening gel. A total of ten teeth whitening treatments was conducted. Duration of each treatment was 5 minutes. Applied electrical current was about 10 milliampere.

Twenty teeth of Group B were treated using commercially available teeth whitening gels without application of electrical current. A total of ten teeth whitening treatments was conducted. The duration of each treatment was 5 minutes.

Whitening of the teeth with an electrically conductive gel assisted by electric current resulted in 35% better average teeth whitening than using commercially available gels without electric current application. Teeth shades were measured by SpectroShade spectrometer commercially available from MHT Optic Research AG, CH-8155 Niederhasli Switzerland. Tooth color shade was evaluated using an established and standardized scale of 16 shades, VITA Classical shade guide (Vident™, Brea, California USA). Teeth shades were measured immediately following completion of each teeth whitening treatment.

Use of electrically conductive gel with application of electric current of about 3-15 milliampere whitened a larger amount of teeth to the whitest A1 color. Electric current assisted tooth whitening treatment was almost 100% more effective than treatment with commercially available gels and without electric current.

Use of electrically conductive gel with application of electric current bleached 90% of the teeth to the whitest A1 shade as compared to only 45% of the teeth treated by commercially available teeth whitening gels and without application of electric current.

100% of the Group A teeth with baseline shade of A3 after treatment with electrically conductive whitening gel and application of electric current have become teeth with A1 shade as compared with 64% of the teeth treated by commercially available dental whitening gel and without application of electric current.

100% of the Group A teeth after treatment with electrically conductive whitening gel and application of electric current have shown whitening of seven shades, as compared with 50% of the teeth treated by commercially available dental whitening gel and without application of electric current.

25% of the Group A teeth treated with electrically conductive whitening gel and application of electric current have shown whitening of ten shades, as compared with 5% of the teeth treated by commercially available dental whitening gel and without application of electric current.

Improvement in whitening of Group B teeth (without application of electric current) has stopped after about 40min of accumulated treatment time, whereas improvement in teeth whitening results of Group A teeth (with application of electric current) continued even after 50min of accumulated treatment time.

### EXAMPLE 5 DENTAL BLEACHING GEL

Further formulations were prepared and tested, wherein the bleaching agent was carbamide peroxide. Furthermore, no binding agent is present.

| Ingredient | Concentration w/w (%) |
|---|---|
| Glycerin/propylene glycol | 25-30 |
| Carbo pol | 1-5 |
| Carbamide peroxide | 25-35 |
| Deionized Water | 30-40 |
| Potassium Nitrate | 2-3 |
| Potassium Hydroxide (50% in water) | 2-3 |
| EDTA (Disodium Ethylenediaminetetraacetic salt) | 0.1-1 |
| Saccharin sodium salt hydrate | 0.2 |
| Peppermint oil | 0.2 |

The formulation was tested as above in example 4. The results are shown in the following table. The formulation using carbamide peroxide was also active when applied and activated with micro-current. This formulation was also stable although it did not comprise a binding agent showing that the essential ingredients as defined in claim 1 are sufficient to prepare a stable and active bleaching gel.

**Tooth bleaching results using the current gel and electric current of 3-15 milliampere**

| **Tooth No.** | **Baseline Shade** | **Tooth shade change** |
|---|---|---|
| T2 | A3 | 7 |
| T4 | A3 | 7 |
| T6 | A3.5 | 10 |
| T8 | A3 | 7 |
| T10 | A3 | 7 |
| T12 | A3 | 7 |
| T14 | C4 | 7 |
| T16 | D3 | 8 |
| T18 | A3 | 7 |
| T20 | A3.5 | 10 |
| T22 | A3 | 7 |
| T24 | C4 | 11 |
| T26 | A3 | 7 |
| T28 | A3.5 | 10 |
| T30 | A3 | 7 |
| T32 | A3 | 7 |
| T34 | A3.5 | 10 |
| T36 | A3 | 7 |
| T38 | A3 | 7 |
| T40 | A3 | 7 |
| **Average** | | **7.85** |

**Tooth bleaching results using a commercially available gel without electric current application**

| **Tooth No.** | **Baseline Shade** | **Tooth shade change** |
|---|---|---|
| T1 | A3 | 7 |
| T3 | A3 | 7 |
| T5 | A3.5 | 4 |
| T7 | A3 | 7 |
| T9 | A3 | 4 |
| T11 | A3 | 7 |
| T13 | D3 | 5 |
| T15 | D3 | 5 |
| T17 | A3 | 4 |
| T19 | A3 | 7 |
| T21 | A3 | 4 |
| T23 | A4 | 5 |
| T25 | A3.5 | 10 |
| T27 | A3 | 4 |
| T29 | A3.5 | 3 |
| T31 | A3 | 7 |
| T33 | A3.5 | 5 |
| T35 | A3 | 7 |
| T37 | A3 | 7 |
| T39 | A3 | 7 |
| **Average** | | **5.8** |

## Claims

1. A teeth bleaching method comprising applying to the teeth to be bleached a layer of gel, wherein the gel comprises (percentages w/w are based on the weight of the final bleaching gel composition):
hydrogen peroxide, wherein the amount of hydrogen peroxide is 1 to 35% w/w, or sodium perborate or sodium percarbonate as a peroxide releasing compound, wherein the amount of the peroxide releasing compound is 1 to 35% w/w;
a cross-linked polyacrylate polymer, wherein the amount of cross-linked polyacrylate polymer is 1 to 5% w/w;
an aqueous vehicle, wherein the amount of glycerine is 20 to 30% w/w; and/or wherein the amount of water is 25 to 50% w/w; and
at least one gel electrical conductivity enhancing ingredient consisting of alkali nitrate in an amount of 1 to 5% w/w and/or alkali hydroxide in an amount of 1 to 5% w/w;
applying to the teeth covered by the layer of gel with electrical conductivity higher than 250 microsiemens/cm electric current of at least 1 milliampere; and
bleaching the teeth.

2. The teeth bleaching method according to claim 1 wherein the amount of alkali hydroxide is such that the gel pH is adjusted to 5-7.

3. The teeth bleaching method according to claim 1 or 2, wherein the alkali hydroxide is potassium hydroxide and/or wherein alkali nitrate is potassium nitrate.

4. The teeth bleaching method according to any one of the preceding claims wherein the gel additionally includes a fluoride in an amount of 0.1 to 1.0% w/w.

5. The teeth bleaching method according to any one of the preceding claims wherein the gel additionally includes a polymeric binder, preferably polyvinylpyrrolidone in an amount of 0.5 to 2.0% w/w.

6. The teeth bleaching method according to any one of the preceding claims wherein the gel additionally includes flavouring ingredients in an amount of 0.1 to 0.5% w/w.

7. The teeth bleaching method according to any one of the preceding claims wherein the flavouring ingredients are at least one of a group consisting of saccharin sodium salt hydrate and peppermint oil and a mix of both of them.

8. The teeth bleaching method according to any one of the preceding claims wherein the gel additionally includes as a complexing agent, disodium ethyelenediamine tetraacetic salt (EDTA) in an amount of 0.1 to 1.0% w/w.

9. Use of a dental bleaching gel as defined in any one of claims 1 to 8 for bleaching teeth by applying the gel to the teeth and applying a micro-current of at least 1 milliampere.

## Patentansprüche

1. Zähnebleichverfahren umfassend Auftragen einer Gelschicht auf die zu bleichenden Zähne, wobei das Gel umfasst (Gewichtsprozentsätze beziehen sich auf das Gewicht der finalen Bleichgelzusammensetzung):
Wasserstoffperoxid, wobei die Menge an Wasserstoffperoxid 1 bis 35 Gew.-% ist, oder Natriumperborat oder Natriumpercarbonat als eine Peroxid freisetzende Verbindung, wobei die Menge der Peroxid freisetzenden Verbindung 1 bis 35 Gew.-% beträgt;
ein quervernetztes Polyacrylatpolymer, wobei die Menge an quervernetztem Polyacrylatpolymer 1 bis 5 Gew.-% ist;
einen wässrigen Träger, wobei die Menge an Glycerin 20 bis 30 Gew.-% ist; und/oder wobei die Wassermenge 25 bis 50 Gew.-% ist; und
mindestens einen die elektrische Leitfähigkeit verstärkenden Gelzusatz, bestehend aus Alkalinitrat in einer Menge von 1 bis 5 Gew.-% und/oder Alkalihydroxid in einer Menge von 1 bis 5 Gew.-%;
Anlegen an die Zähne, die mit der Gelschicht mit einer elektrischen Leitfähigkeit größer als 250 Mikrosiemens/cm bedeckt sind, eines elektrischen Stroms von mindestens 1 Milliampere; und
Bleichen der Zähne.

2. Zähnebleichverfahren nach Anspruch 1, worin die Menge an Alkalihydroxid derart ist, dass der Gel-pH-Wert auf 5-7 eingestellt ist.

3. Zähnebleichverfahren nach Anspruch 1 oder 2, wobei das Alkalihydroxid Kaliumhydroxid ist und/oder wobei das Alkalinitrat Kaliumnitrat ist.

4. Zähnebleichverfahren nach irgendeinem der vorhergehenden Ansprüche, wobei das Gel zusätzlich ein Fluorid in einer Menge von 0,1 bis 1,0 Gew.-% einschließt.

5. Zähnebleichverfahren nach irgendeinem der vorhergehenden Ansprüche, worin das Gel zusätzlich ein polymeres Bindemittel einschließt, vorzugsweise Polyvinylpyrrolidon, in einer Menge von 0,5 bis 2,0 Gew.-%.

6. Zähnebleichverfahren nach irgendeinem der vorhergehenden Ansprüche, worin das Gel zusätzlich Geschmacksbestandteile in einer Menge von 0,1 bis 0,5 Gew.-% einschließt.

7. Zähnebleichverfahren nach irgendeinem der vorhergehenden Ansprüche, worin die Geschmacksbestandteile mindestens eines aus der Gruppe bestehend aus Saccharinnatriumsalzhydrat und Pfefferminzöl und einer Mischung von beiden davon sind.

8. Zähnebleichverfahren nach irgendeinem der vorhergehenden Ansprüche, worin das Gel zusätzlich als ein Komplexierungsmittel Dinatriumethylendiamintetraessigsäuresalz (EDTA) in einer Menge von 0,1 bis 1,0 Gew.-% einschließt.

9. Verwendung eines Zahnbleichgels wie in irgendeinem der Ansprüche 1 bis 8 definiert zum Bleichen von Zähnen durch Auftragen des Gels auf die Zähne und Anbringen eines Mikrostroms von mindestens 1 Milliampere.

## Revendications

1. Procédé de blanchiment des dents comprenant l'application sur les dents à blanchir d'une couche de gel, le gel comprenant (les pourcentages p/p sont basés sur le poids de la composition finale de gel de blanchiment) :
peroxyde d'hydrogène, la quantité de peroxyde d'hydrogène étant de 1 à 35 % p/p, ou perborate de sodium ou percarbonate de sodium en tant que composé de libération de peroxyde, la quantité du composé de libération de peroxyde étant de 1 à 35 % p/p ;
un polymère polyacrylate réticulé, la quantité de polymère polyacrylate réticulé étant de 1 à 5 % p/p ;
un véhicule aqueux, la quantité de glycérol étant de 20 à 30 % p/p ; et/ou la quantité d'eau étant de 25 à 50 % p/p ; et
au moins un ingrédient augmentant la conductivité électrique du gel, consistant en nitrate alcalin dans une quantité de 1 à 5 % p/p et/ou en hydroxyde alcalin dans une quantité de 1 à 5 % p/p ;
appliquer sur les dents recouvertes par la couche de gel avec une conductivité électrique supérieure à 250 microsiemens/cm un courant électrique d'au moins 1 milliampère ; et
blanchir les dents.

2. Procédé de blanchiment des dents selon la revendication 1, dans lequel la quantité d'hydroxyde alcalin est telle que le pH du gel est ajusté à 5-7.

3. Procédé de blanchiment des dents selon l'une des revendications 1 ou 2, dans lequel l'hydroxyde alcalin est l'hydroxyde de potassium et/ou dans lequel le nitrate alcalin est le nitrate de potassium.

4. Procédé de blanchiment des dents selon l'une quelconque des revendications précédentes, dans lequel le gel comprend en outre un fluorure dans une quantité de 0,1 à 1,0 % p/p.

5. Procédé de blanchiment des dents selon l'une quelconque des revendications précédentes, dans lequel le gel comprend en outre un liant polymère, de préférence la polyvinylpyrrolidone dans une quantité de 0,5 à 2,0 % p/p.

6. Procédé de blanchiment des dents selon l'une quelconque des revendications précédentes, dans lequel le gel comprend en outre des ingrédients aromatisants dans une quantité de 0,1 à 0,5 % p/p.

7. Procédé de blanchiment des dents selon l'une quelconque des revendications précédentes, dans lequel les ingrédients aromatisants sont au moins l'un d'un groupe consistant en l'hydrate de sel de sodium de saccharine et l'essence de menthe poivrée et un mélange des deux.

8. Procédé de blanchiment des dents selon l'une quelconque des revendications précédentes, dans lequel le gel comprend en outre comme agent complexant, le sel éthylènediamine tétra-acétique disodique (EDTA) dans une quantité de 0,1 à 1,0 % p/p.

9. Utilisation d'un gel de blanchiment des dents selon l'une quelconque des revendications 1 à 8 pour le blanchiment des dents par application du gel sur les dents et application d'un micro-courant d'au moins 1 milliampère.
